# FASCICULE DE BREVET EUROPEEN

(11) **EP 1 579 354 B1**
(45) Date de publication et mention de la délivrance du brevet: **24.07.2019**
(21) Numéro de dépôt: 03814510.8
(22) Date de dépôt: 30.12.2003
(51) Int. Cl.: G06Q 50/22, G16H 50/50

(54) **PROCEDE DE SIMULATION BIOMECANIQUE D'UN ENSEMBLE D' ARTICULATIONS OSSEUSES**
VERFAHREN ZUR BIOMECHANISCHEN SIMULATION EINER GRUPPE VON GELENKEN
METHOD FOR BIOMECHANICALLY SIMULATING A SET OF OSSEOUS JOINTS

(30) Priorité: 30.12.2002 FR 0216846
(43) Date de publication de la demande: 28.09.2005
(73) Titulaire: AXS Medical, 76068 Le Havre (FR)
(72) Inventeur: ELBAROUDI, Fouad, 33600 PESSAC (FR)
(74) Mandataire: Ipside
(86) Numéro de dépôt international: PCT/FR2003/003943
(87) Numéro de publication internationale: WO 2004/061721

(56) Documents cités:
- WO-A-02/097735
- GUILLAUME MUSSEAU: "Personnalisation géométrique d'un modèle de colonne vertébrale pour un simulateur d'opération du rachis" RAPPORT DE STAGE, 20 septembre 2002 (2002-09-20), pages 1-47, XP002301694
- AUBIN C-E ET AL: "MORPHOMETRIC EVALUATIONS OF PERSONALISED 3D RECONSTRUCTIONS AND GEOMETRIC MODELS OF THE HUMAN SPINE" MEDICAL AND BIOLOGICAL ENGINEERING AND COMPUTING, PETER PEREGRINUS LTD. STEVENAGE, GB, vol. 35, no. 6, 1 novembre 1997 (1997-11-01), pages 611-618, XP000723473 ISSN: 0140-0118
- ANDRE B ET AL: "OPTIMIZED VERTICAL STEREO BASE RADIOGRAPHIC SETUP FOR THE CLINICAL THREE-DIMENSIONAL RECONSTRUCTION OF THE HUMAN SPINE" août 1994 (1994-08), JOURNAL OF BIOMECHANICS, PERGAMON PRESS, NEW YORK, NY, US, PAGE(S) 1023-1025,1027 , XP000997655 ISSN: 0021-9290 en particulier: chapitres: Introduction, Validation test on the calibration object,, Three-dimensional reconstructions of the spine for 0-5 to 0-30 vertical setups le document en entier
- HAYASAKA T ET AL: "A MRI based semi-automatic modeling system for computational biomechanics simulation" MEDICAL IMAGING AND AUGMENTED REALITY, 2001, INTERNATIONAL WORKSHOP ON 10-12 JUNE 2001, 10 juin 2001 (2001-06-10), pages 282-285, XP010547543
- GRÉALOU L ET AL: "Rib cage surgery for the treatment of scoliosis: a biomechanical study of correction mechanisms." JOURNAL OF ORTHOPAEDIC RESEARCH: OFFICIAL PUBLICATION OF THE ORTHOPAEDIC RESEARCH SOCIETY. UNITED STATES SEP 2002, vol. 20, no. 5, septembre 2002 (2002-09), pages 1121-1128, XP002256910 ISSN: 0736-0266

## Description

La présente invention se rapporte au domaine des logiciels de simulation biomédicale.

La présente invention se rapporte plus particulièrement à un procédé de simulation biomécanique d'un ensemble d'articulations osseuses d'un patient, et notamment du rachis. Ce procédé permet de calculer, estimer et visualiser les conséquences d'une opération chirurgicale sur une articulation. Le système a été mis au point pour les opérations de la colonne vertébrale et en particulier les techniques de stabilisation de la colonne vertébrale. D'une manière générale, le système permet d'informer le chirurgien sur l'état de l'équilibre et la répartition des efforts dans la colonne du patient avant et après la simulation.

L'art antérieur connaît déjà, par la demande de brevet américain US 5 995 738, un dispositif et une méthode pour faciliter l'implantation de composants artificiels dans les articulations. L'invention décrit des dispositifs et des méthodes destinés à déterminer une position d'implant pour au moins un composant artificiel dans une articulation et à faciliter son implantation. L'invention comprend la création d'un modèle de l'articulation du patient et la création d'un modèle du composant à implanter. Les modèles créés sont utilisés pour simuler le mouvement de l'articulation du patient selon la position du composant. Ce document de l'art antérieur propose donc un moyen de simulation physique et non virtuel pour analyser les mouvements d'une articulation et d'un implant.

Il est proposé, dans la demande de brevet américain US 6 205 411, un planificateur de chirurgie assistée par ordinateur et un système de guidage intra-opératif. L'invention concerne un appareil destiné à faciliter l'implantation d'un composant artificiel dans une articulation. L'appareil comprend un prédicteur géométrique et un simulateur biomécanique du mouvement pré-opératif, c'est-à-dire qu'une série de simulations est effectuée sur l'implant et l'articulation avant de procéder à l'opération.

Il est proposé, dans la demande de brevet américain US 5 625 577, une méthode informatique d'analyse de mouvement utilisant la dynamique. L'invention décrit une méthode permettant d'analyser et d'afficher les mouvements d'un être humain. Le corps du sujet est divisé en une pluralité de segments reliés entre eux par des articulations. Une fois le corps modélisé ainsi, il est possible de simuler et d'analyser le mouvement du sujet.

Il est également proposé, dans la demande de brevet PCT WO 99/06960, un système et procédé permettant de définir et d'utiliser des comportements destinés à des chaînes articulées dans des animations par ordinateur. En l'occurrence, on définit pour la chaîne articulée au moins une commande telle qu'une commande de forme ou une commande de plan de résolution, et on utilise cette commande comme une contrainte reprise par le moteur d'animation pour l'animation de la chaîne avec une cinématique inverse. Chaque commande comporte au moins deux clés, chaque clé comprenant un couple constitué d'un vecteur de sens effecteur et d'une contrainte associée. Dans le cas des clés de commande de forme, les contraintes associées comprennent une liste d'orientations préférentielles des membres du corps. Dans le cas des clés de commande de plan de résolution, les contraintes associées comportent une orientation préférentielle du plan de résolution. Quels que soient les buts assignés, les clés de commande sélectionnées subissent une interpolation mettant en oeuvre des pondérations appropriées visant à l'obtention d'une contrainte résultante à faire utiliser par le moteur d'animation.

L'art antérieur connaît également, par la demande de brevet PCT WO 99/60939, un système chirurgical interactif assisté par ordinateur pour aider le chirurgien à positionner des implants dans le fémur ou des vis dans les pédicules des vertèbres. Ces systèmes apportent une assistance à la navigation. Cependant, c'est une assistance axée principalement sur le positionnement, le repérage et le guidage des ancillaires. Cette technique de navigation chirurgicale est assez répandue, mais ne permet pas de répondre à toutes les interrogations du chirurgien. En effet, l'utilisation de ces systèmes permet de définir la trajectoire optimale de la vis pédiculaire, mais ne permet pas de savoir par exemple, si elle a été placée sur la bonne vertèbre.

Dans la publication scientifique « Morphometric evaluations of personalised 3D reconstructions and geometric models of the human spine » Aubin C-E et al - Medical and biological engineering and computing, Peter Perenigrus Ltd. Stevenage, GB - 01/11/1997, plusieurs méthodes de reconstructions géométriques sont évaluées. Dans la conclusion de cette publication scientifique, les auteurs indiquent qu'une des méthodes évaluées est optimale. L'étude du Dr Aubin confirme qu'une reconstruction géométrique d'une colonne vertébrale à partir de plusieurs radiographies constitue une approche pertinente et reproductible pour l'étude et l'évaluation des dysfonctionnements du rachis ainsi que pour sa modélisation par la méthode des éléments finis. Cette publication ne porte aucunement sur les aspects de personnalisation mécanique in vivo (mobilité et anthropométrie), ni sur la simulation de stratégies opératoires, ni sur leurs méthodes d'optimisation par approche biomécanique. Enfin l'approche mise en oeuvre dans la présente invention associant plusieurs techniques (personnalisation géométrique, mobilité et anthropométrie in vivo, analyse numérique, critères d'équilibre, méthodes de recalage entre données internes et externes, calculs des efforts) n'y est ni abordée ni suggérée.

Enfin, l'art antérieur connaît, par la publication scientifique « A MRI based semi-automatic modeling system for computational biomechanics simulation » Hayasaka T et al - Medical imaging and augmented reality, 2001, International workshop on 10-12 june 2001 - 10/06/2001, un procédé de génération semi-automatique d'un maillage. Le procédé décrit dans ce document s'applique au système cardio-vasculaire, c'est-à-dire aux tissus mous et non aux articulations osseuses comme la présente invention. A partir d'un modèle issu d'une base de données et d'une image IRM, un algorithme crée un modèle spécifique en vue d'une étude (nous présumons de la tenue mécanique du tissu) basé sur la méthode des éléments finis. L'auteur cite par ailleurs des difficultés restant à résoudre en vue de créer un maillage utilisable. La simulation mécanique des tissus mous qui reste à ce jour du domaine de la recherche fondamentale, est confrontée à la difficulté de les caractériser mécaniquement in vitro et encore plus in vivo. Cette étude ne porte pas sur la simulation d'un ensemble d'articulations osseuses. Elle porte sur un procédé de génération semi-automatique d'un maillage.

Ces deux publications scientifiques traitent de problématiques différentes de celle de la présente invention. En particulier, elles ne mentionnent nullement d'étape de personnalisation d'un modèle numérique par particularisation des paramètres d'interaction [de mobilités ou caractéristiques de rigidités] de chacune des articulations reliant lesdits corps rigides en fonction des caractéristiques constatées sur le patient. Cette étape est fondamentale dans la présente invention.

La présente invention entend remédier aux inconvénients de l'art antérieur en permettant de simuler une opération de correction locale ou globale de courbures de la colonne vertébrale ou de pose d'implant vertébral à partir d'images radiographiques, de séries d'acquisitions de caractéristiques mesurées in vivo sur le patient et d'une base de données d'implants. La présente invention permet de plus de simuler l'état de la colonne juste après l'opération chirurgicale.

Bien entendu, la présente invention ne s'applique pas uniquement au rachis. Elle s'applique également à d'autres articulations osseuses comme le genou.

L'invention consiste en un système chirurgical assisté par ordinateur, permettant au chirurgien de simuler, en préopératoire, les effets sur le patient de la chirurgie correctrice qu'il envisage de mettre en application. Ce système lui permettant de simuler ainsi plusieurs stratégies opératoires, il offre au chirurgien un outil d'aide au choix de la stratégie opératoire offrant le meilleur compromis entre la stabilisation et la mobilité.

A cet effet, la présente invention concerne dans son acception la plus générale un procédé de construction d'un modèle numérique d'un ensemble d'articulations osseuses d'un patient, notamment du rachis, chaque articulation osseuse comportant deux os reliés par une articulation, assisté par ordinateur et dans lequel :
- l'ordinateur comporte un modèle numérique tridimensionnel de référence de l'ensemble d'articulations osseuses représenté au moins en partie par des corps rigides modélisant les os et reliés par des articulations, dans une position de référence ;
   - la géométrie dudit modèle numérique [position relative dans l'espace de chacun desdits corps rigides] est personnalisée par des données géométriques spécifiques issues directement ou indirectement d'images médicales du patient dans ladite position de référence [par exemple des radiographies du patient] ;
   - ledit modèle numérique est personnalisé mécaniquement par des modifications des paramètres d'interaction [de mobilités ou caractéristiques de rigidité] de chacune des articulations reliant lesdits corps rigides en fonction de caractéristiques constatées sur le patient ;
la personnalisation mécanique du modèle numérique consiste à :
- acquérir les positions dans l'espace d'une partie au moins des corps rigides, et à procéder à une interpolation pour déterminer la position calculée des autres corps rigides pour construire une table numérique comportant les positions relatives de chacun des corps rigides du modèle numérique ;
- mesurer des positions relatives correspondant à une position générale d'équilibre du patient [autre que la position de référence] et résultant de l'application d'au moins une contrainte déterminée sur ledit patient ;
- calculer, pour chaque couple de corps rigides, des fonctions analytiques permettant d'approximer les paramètres d'interaction [de mobilités ou caractéristiques de rigidité], et qui appliquées au modèle numérique depuis la position de référence conduisent aux positions relatives mesurées correspondant à la position d'équilibre résultant de l'application de l'au moins une contrainte sur le patient.

De préférence, le modèle numérique est défini par des paramètres de positions géométriques des corps rigides et par des paramètres de rigidité des articulations reliant les corps rigides.

Avantageusement, l'étape de représentation du résultat d'une contrainte consiste à recalculer le modèle personnalisé [en position d'équilibre] résultant d'un ensemble de contraintes [par exemple implantation d'une prothèse ou d'un implant] comprenant au moins une contrainte statique exercée sur au moins deux corps rigides, et imposant un positionnement relatif avec une mobilité ou une raideur différente de celle correspondant à la loi de comportement.

Selon une variante, l'étape d'enregistrement du modèle numérique de l'ensemble d'articulations standard consiste à définir une alternance de corps rigides et d' articulations, et à définir pour chacun des couples de corps un ensemble de paramètres numériques caractérisant la mobilité ou raideur globale résultant de l'action de l'ensemble des éléments intercalaires [par exemple disques intervertébraux] et des éléments de liaison [par exemple ligaments] ayant un effet sur les paramètres d'interaction [rigidités] entre les deux corps.

Selon un mode de mise en oeuvre particulier, l'étape de personnalisation consiste à acquérir au moins une image de l'ensemble d'articulations d'un patient donné, à extraire de ladite image les informations nécessaires à la construction d'un modèle réel par reconnaissance de la position des articulations visibles dans ladite image, et de modifier le modèle standard en fonction dudit modèle réel.

Avantageusement, l'étape d'enregistrement d'un modèle numérique consiste à définir un ensemble standard de données numériques comprenant pour chacune des articulations représentées sous la forme d'un corps rigide :
- un premier descripteur géométrique de position de référence, correspondant à la géométrie de l'ensemble d'articulation pour un patient « standard » dans une position «de référence», ledit descripteur étant déterminé pour chaque corps rigide de façon relative par rapport à un corps adjacent ;
- un deuxième descripteur mécanique d'interaction avec chacun des corps adjacents, ledit descripteur mécanique étant représentatif de la loi de comportement lorsque l'ensemble d'articulation est soumis à l'action d'au moins une contrainte extérieure ;
l'étape de personnalisation consistant à modifier ledit ensemble standard de données par des données personnalisées.

Selon un mode de mise en oeuvre particulier, le procédé comporte en outre une étape de recalage consistant à mettre en correspondance des données d'images radios et des données d'acquisition externe, cette étape se décomposant en deux sous-étapes :
- recalage de la reconstruction radio par rapport à la courbe 3D issue de données d'acquisition externe dans la même position ;
- détermination de la répartition des points de la courbe 3D associés aux vertèbres, positionnés dans le repère de Stokes et leur tangente associée.

On comprendra mieux l'invention à l'aide de la description, faite ci-après à titre purement explicatif, d'un mode de réalisation de l'invention, en référence aux figures annexées :
- la figure 1 illustre l'architecture de la personnalisation mécanique du modèle ;
- la figure 2 représente l'architecture générale du simulateur selon un mode de réalisation de l'invention ;

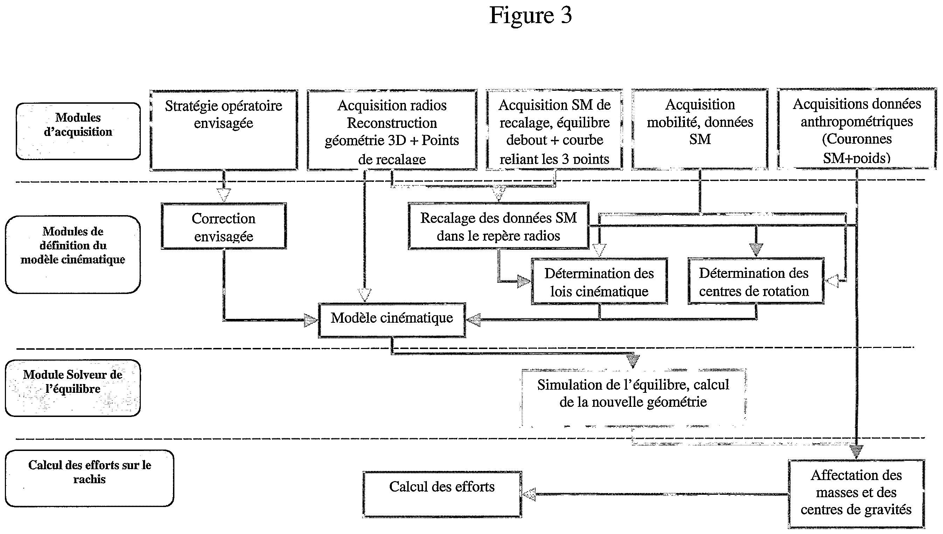

- la figure 3 représente l'architecture du modèle selon un mode de mise en oeuvre de l'invention ;

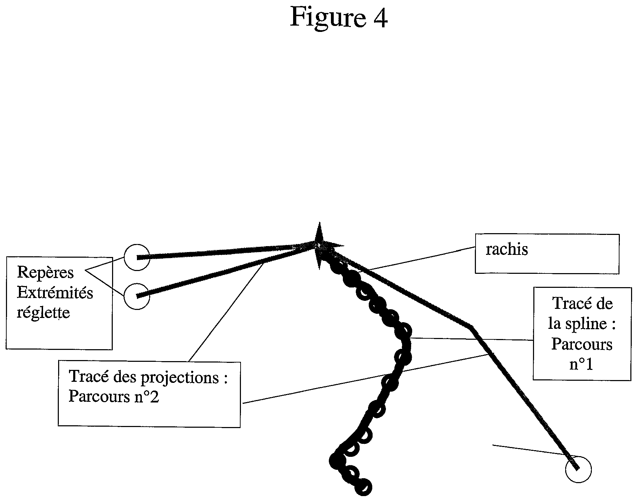

- la figure 4 illustre la mise en oeuvre du procédé de recalage ;
- la figure 5 illustre la définition des angles intervertébraux ;
- la figure 6 représente un exemple des relations entre les angles intervertébraux dans le plan frontal (abscisse en ° et ordonnée en °) ;
- la figure 7 illustre le calcul des centres de rotation ;
- la figure 8 représente un exemple de calcul des centres de rotation sur un sujet scoliotique ;
- la figure 9 illustre l'interpolation des couronnes ; et
- la figure 10 représente un modèle de répartition des masses dans le tronc.

L'invention se distingue des produits connus de guidage chirurgicaux, utilisés pour assister le chirurgien pendant son opération. En effet, l'invention consiste notamment à modéliser la colonne vertébrale de l'individu qui va être opéré, de simuler la pose de l'implant ou de prothèse et de calculer la position d'équilibre de l'individu une fois l'implant ou la prothèse posé.

Le traitement des données étant logiciel, l'invention concerne aussi l'architecture logicielle mise en place pour la réalisation de l'architecture fonctionnelle. La structure logicielle comprend plusieurs serveurs de bases de données : une base de données utilisateurs, une base de données vertèbres, une base de données patients et une base de données implants. Les données contenues dans ces bases sont interrogées et mises à jour par différents utilisateurs afin de construire le modèle 3D de la colonne du patient puis de simuler les conséquences de la mise en place d'un implant.

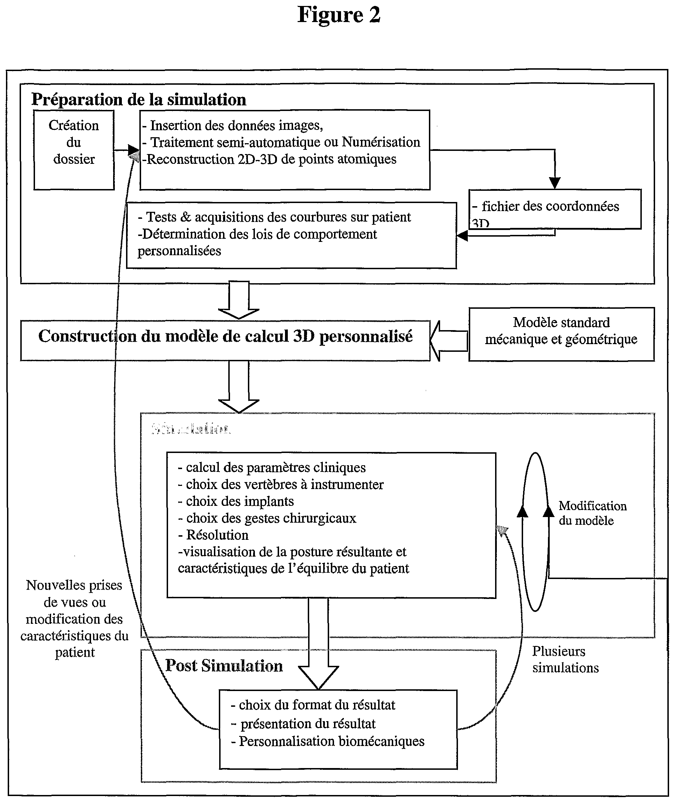

Le système selon l'invention est un simulateur des conséquences biomécaniques et cinématiques de l'acte chirurgical des traitements des pathologies rachidiennes sur la morphologie du patient. La Figure 2 représente l'architecture générale du simulateur.

Il doit permettre à un chirurgien d'optimiser et améliorer la planification de son traitement. Ce simulateur pourra lui apporter une meilleure connaissance des propriétés géométriques et mécaniques des divers tissus de la colonne vertébrale. Il pourra aussi tester différentes approches de son geste pour permettre une correction optimale.

Il doit répondre à un besoin de santé, puisque la tendance est à la recherche du confort, de la sécurité, de la qualité et de la fiabilité. L'allégement des traitements médicaux se traduit par une meilleure qualité de vie post-opératoire et en particulier par des hospitalisations de fréquence moindre et de durée plus courte.

Enfin, il peut également avoir des retombées dans le domaine de l'enseignement pour l'apprentissage des futurs chirurgiens.

Le simulateur est un outil d'aide à l'analyse de la faisabilité du geste. Il permet de simuler l'équilibre préopératoire, les efforts inter-segmentaires correspondants tenant compte de l'effet des rigidités musculaires et ligamentaires, appréhender l'évolution de cet équilibre et de ces efforts en post-opératoire en fonction des courbures introduites par le chirurgien lors de l'opération. Le chirurgien devra indiquer en entrée la déformée voulue de la zone instrumentée. Pour cela une approche dite « globale » sera utilisée.

Deux radiographies sont nécessaires pour la réalisation d'une simulation. Les images à ajouter au système peuvent être :
- le fichier résultat de la numérisation (scan) d'une radiographie traditionnelle ;
- un fichier fourni par un autre appareil de radiologie (radiographie numérique).

L'alimentation du système peut être manuelle (un utilisateur va fournir les fichiers d'images au système) ou automatique, les images sont stockées directement par les appareils de radiologie et récupérées via un réseau intranet/internet.

Deux sortes de numérisations seront possibles :
- numérisation manuelle : des points spécifiques doivent être repérés manuellement par un acteur sur ces images ;
- numérisation semi-automatique.

Les points spécifiques détectés par la numérisation vont permettre de calculer les coordonnées 3D des vertèbres (données géométriques).

Les données géométriques issues des images numériques vont permettre de construire un modèle de la colonne du patient en trois dimensions. Ce modèle résulte de l'adaptation d'un modèle 3D standard, prédéfini dans le système, aux caractéristiques géométriques du patient.

L'utilisateur doit pouvoir visualiser le modèle 3D du rachis selon les plans frontal, sagittal et apical ; de plus, il doit pouvoir le comparer aux radiographies utilisées pour sa construction.

Les caractéristiques mécaniques du patient (résultats issus d'acquisitions (tests cliniques)) permettent de personnaliser le modèle. Pour cette opération, un modèle géométrique standard de la colonne vertébrale est enrichi par les données mécaniques personnelles du patient concerné.

Les caractéristiques biomécaniques de la colonne du patient (angle de scoliose, rotation axiale, etc.) sont calculées à partir des radiographies du patient (avant la première simulation), ou à partir du modèle résultant d'une simulation. Certains paramètres pourront être pré-calculés (pente sacrée, angle d'incidence, gîte sagittale, courbures rachidiennes)sur les radiographies.

Lors de la simulation, l'utilisateur doit avoir la possibilité de choisir des segments de colonne sur lesquels il impose des déplacements. Lors de la simulation de l'équilibre, l'utilisateur va pouvoir entrer les valeurs de certains paramètres cliniques qu'il souhaite simuler pour un segment donné. L'utilisateur doit pouvoir consulter les mobilités ou rigidités du rachis, la graduation sera normée. Le type de la tige utilisée pour déformer un segment peut être choisi parmi plusieurs propositions (rigidité, diamètre, etc.).

A la suite d'une manipulation du modèle par l'utilisateur, le logiciel doit vérifier la validité des actions effectuées, et prévenir l'utilisateur de toute incohérence (valeur impossible à obtenir).

Le simulateur doit afficher les nouvelles courbures du modèle et la nouvelle position (stature) de l'équilibre du patient. Il doit être possible de comparer la (les) courbure(s) avec la (les) courbure(s) initiale(s) (avec le modèle 3D ou la radiographie).

L'évolution relative des efforts intervertébraux est quantifiée et représentée graphiquement.

L'utilisateur doit pouvoir comparer les rigidités du rachis avant et après la simulation.

Les efforts résultants dans la tige suite aux manipulations effectuées doivent indiquer si la tige va se déformer.

Des dossiers patients, contenant les informations concernant la simulation, doivent pouvoir être créés par un utilisateur et maintenus à jour par le système, (sauvegarde de plusieurs simulations dans un même dossier). De plus, le système doit pouvoir retirer ou saisir les informations concernant un patient (âge, poids, taille, etc.) dans des systèmes de données externes, sous réserve de leur existence.

Les droits et profils utilisateurs vont permettre de différencier les fonctionnalités disponibles pour chaque utilisateur. En fonction de leurs droits et profils, les utilisateurs auront accès à des interfaces graphiques différentes. De plus, un système de préférence pourra être mis en place pour chaque utilisateur (position des menus, page d'ouverture etc.), ainsi qu'un mécanisme de traçabilité, qui permettra de suivre les évolutions d'un dossier patient.

A tout moment, et en fonction de ses droits, un utilisateur peut avoir accès aux données d'entrées, c'est-à-dire visualiser, remplacer ou modifier les images utilisées pour la construction du modèle, les points numérisés ou les opérations effectuées pour le traitement semi-automatique de ces images, les implants placés sur le modèle, les caractéristiques mécaniques du patient...

L'historique d'une simulation représente la totalité des actions effectuées sur un patient (paramètres de la simulation). L'utilisateur doit pouvoir revenir sur une action passée (changer une radiographie, ressaisir les points pour la numérisation, calculer de nouveaux paramètres cliniques, ou modifier les actions faîtes au cours de la simulation)

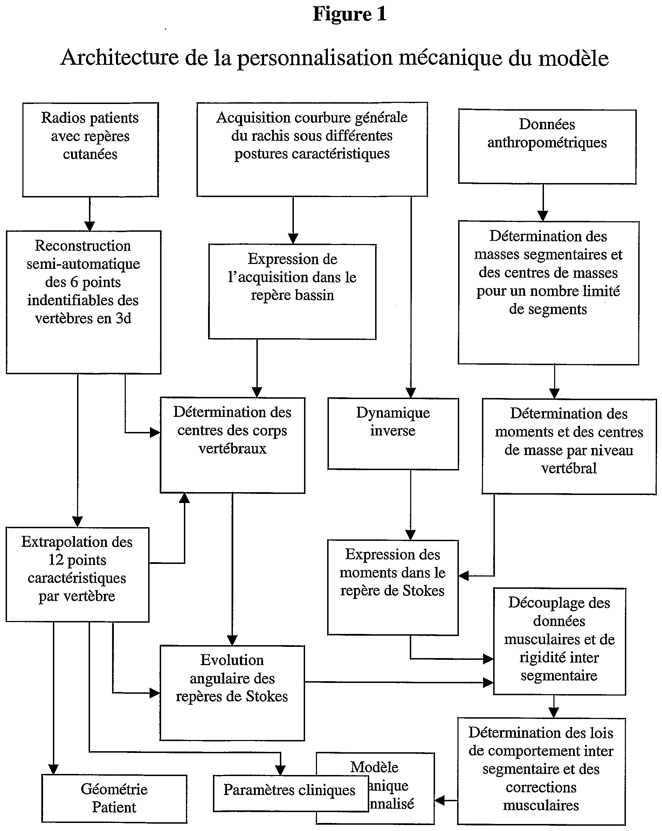

La Figure 1 représente l'architecture de la personnalisation mécanique du modèle.

La personnalisation mécanique du modèle est basée sur trois types de données :
- les radiographies du patient avec des repères cutanés ;
- l'acquisition de la courbure générale du rachis sous différentes postures caractéristiques ;
- des données anthropométriques.

A partir de ces données, des traitements utilisant les lois de la mécanique nous permettent d'obtenir :
- la géométrie du patient ;
- les paramètres cliniques ;
- le modèle mécanique personnalisé.

La numérisation des radiographies du patient nous permet d'obtenir une précision de six points par vertèbre. En effectuant une extrapolation, on arrive à une précision de douze points par vertèbre.

L'étape suivante est l'acquisition de la courbure générale du rachis sous différentes postures caractéristiques.

Pour ce faire, lors d'un examen clinique, le patient étudié est soumis à une série de tests au cours desquels sera évaluée la ligne générale de la colonne au niveau dorsal. Le positionnement du bassin et des épaules sera nécessaire pour définir les orientations des extrémités de la colonne.

Le patient devra être, éventuellement, maintenu au niveau du bassin pour limiter l'intervention des membres extérieurs dans l'établissement de son équilibre général, il sera effectué une acquisition à l'aide d'un appareil, des positions dans l'espace de repères cutanés identifiables liés aux vertèbres scoliotiques par rapport à un référentiel connu.

Une acquisition sera effectuée patient au repos dans des conditions aussi proches que possible des conditions de prise de la radiographie calibrée patient debout au repos. Cette acquisition sera utilisée pour déterminer la ligne des centres des corps vertébraux à partir de la ligne générale de la colonne. Ceci est rendu possible par le fait que la radio calibrée peut donner les coordonnées de tous ces points grâce aux repères plombés. Le positionnement des vertèbres peut donc être déterminé en fonction des repères cutanés pour une position donnée, debout, au repos. Il sera ensuite établi une transformation corrigée entre les repères cutanés et le positionnement des vertèbres prenant en compte l'influence de la cinématique des vertèbres entre elles lors des mouvements du patient.

A partir des données anthropométriques, on détermine les masses segmentaires et les centres de masses, permettant ensuite de déterminer les moments et les centres de masse par niveau vertébral.

L'action musculaire sera dissociée de l'action inter segmentaire.

Les données disponibles dans la littérature permettent d'évaluer qualitativement la forme des lois de comportement admissibles pour le modèle.

Les lois de comportement doivent répondre aux exigences suivantes :
- la plupart des lois doivent suivre des comportements impairs ;
- le comportement asymptotique doit être assuré ;
- les phénomènes de couplage sont pris en comptes ;
- la pertinence et la simplicité de calcul.

Les lois de comportement devront être recalculées pour chaque niveau vertébral et pour chaque patient afin de prendre en compte les singularités dues aux pathologies étudiées.

Selon une variante de l'invention, on met en correspondance les radios avec les acquisitions externes. La mise en correspondance se passe en deux étapes :
- Le recalage de la reconstruction radio par rapport à la courbe 3D issue de donnes d'acquisition externe dans la même position.
- La détermination de la répartition des points de la courbe 3D associés aux vertèbres, positionnés dans le repère de Stockes et leur tangente associées.
Lors des acquisitions radio et de données externes, trois repères spatiaux sont également enregistrés. Les deux extrémités de la réglette, et une bille en plomb (point bas), placée en bas de la colonne.

Ces trois points dont on connaît la position relative, à la fois dans le repère de reconstruction radio et dans le repère des données d'acquisition externes, permettent de recaler les deux enregistrements dans le même repère, celui de la radio.

Les trois points sont suffisants à déterminer un repère commun dans lequel l'ensemble des données est connu, ce qui permet donc de définir la matrice de passage entre les deux enregistrements.

Compte tenu des erreurs d'acquisition possible, rotation du poignet involontaire, rugosité de la peau variable le long de l'enregistrement, trois autres recalages sont également effectués :
1) on compare la distance entre le point bas de la réglette et le point bas placé sur la peau, pour la radio et l'acquisition externe. La différence entre les deux enregistrements quantifie le trajet effectué par la spline sur la peau et donc réajuste l'acquisition vis-à-vis de la pression exercée par l'opérateur sur la peau du sujet.
2) Cet ajustement est complété par un ajustement des longueurs des acquisitions externes de telle manière à garantir le fait que les quatre acquisitions partent du même point.
3) Lors de l'acquisition, suivant le positionnement de l'opérateur, il est fréquent de constater un biaisement de l'acquisition vers la gauche ou la droite. Ce décalage est corrigé en redressant les acquisitions en contraignant l'acquisition allant jusqu'au point bas de respecter la jonction entre le point concourant défini au 2 et le point bas défini sur la radio.

Suite à ces trois recalages, il est possible d'avoir une confiance certaine dans le recalage effectué, corrigé des erreurs.

Répartition des vertèbres sur la courbe 3D :
À partir du recalage entre les radios, la deuxième étape consiste à placer les vertèbres sur la spline. On considère que le point de correspondance entre la vertèbre et la spline est le point d'intersection du repère de Stockes (plan XY) avec celle-ci.

Pour les vertèbres du bas de la colonne, la distance importante entre la colonne et la surface du dos, ainsi que la forte inclinaison des vertèbres provoquent des positionnements non successifs des vertèbres sur la Courbe 3D. Sur cette portion d'enregistrement, on applique la démarche inverse, on place des points équidistants puis on définit leurs positions dans le repère de Stokes de la vertèbre correspondante.

On enregistre également les tangentes de la Courbe 3D aux points considérés de manière à pouvoir par la suite repositionner le modèle.

Cette méthode permet de définir également sur les enregistrements d'acquisitions externes la position du sacrum et l'angle du bassin avec la verticale, appelé angle sacré.

Même si ces parties anatomiques ne font pas parties de la reconstruction à partir de la radio, ils sont utiles pour définir l'équilibre du rachis.

On suppose ensuite que la répartition des vertèbres sur toutes les acquisitions externes reste la même sur toutes les acquisitions, ce qui permet de positionner les vertèbres dans toutes les positions.

Notons quelques remarques sur l'analyse des données :
∘ L'étude se fait sur les deux plans frontal et sagittal séparément. En effet, le choix a été pris d'étudier dans un premier temps la colonne vertébrale de manière découplée. C'est pourquoi les mouvements demandés aux patients sont des inflexions latérales pour l'analyse du plan frontal et des flexions/extensions pour l'analyse du plan sagittal.

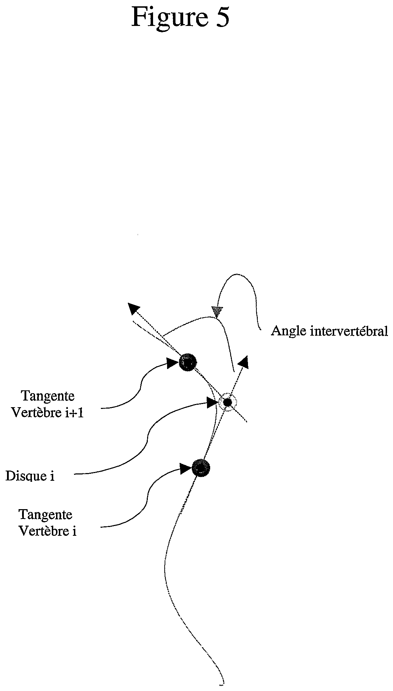

Le calcul des angles intervertébraux est illustré sur la figure 5.

Le but est de trouver une relation liant les angles intervertébraux. Pour cela, un module d'analyse a été ajouté, traçant un angle intervertébral en fonction de celui qui se trouve « en dessous » lorsque l'on parcourt la colonne de bas en haut.

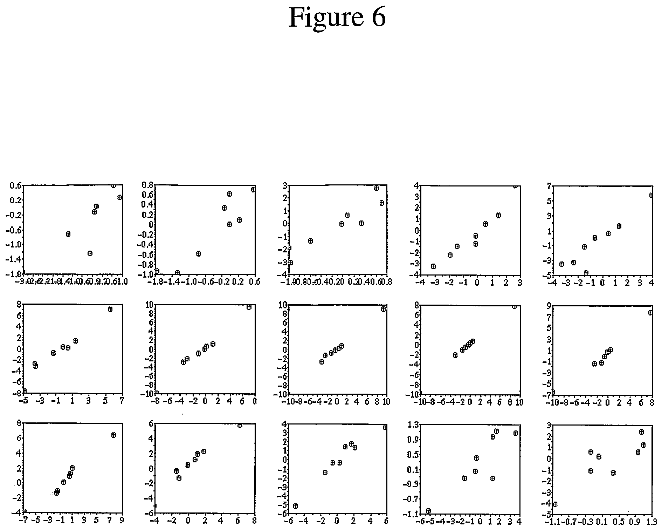

Exemple de tracé des relations entre les angles intervertébraux (le premier graphique représente *θ*_{(*C*7*,T*1*)*/(*T*1*,T*2)} haut à gauche, puis on parcourt la colonne en descendant dans le sens de lecture classique) :La figure 6 illustre un exemple des relations entre les angles intervertébraux dans le plan frontal (abscisse en ° ; ordonnée en °)

Les conclusions de ces nuages de points sont les mêmes pour tous les sujets. La distribution est considérée linéaire. Le modèle est donc bâtit sur ces constatations :
On définit l'équation de régression sur les points à chaque niveau intervertébral. A chaque équation on adjoint des bornes caractérisant la nature bornée de la mobilité d'une vertèbre par rapport à celle qui se trouve en dessous. On fait alors l'hypothèse que les mouvements d'inflexion latérale et de flexion/extensions maximales définissent ces butées.

Cette approche a été validée en analysant un certain nombre de sujets sains. Pour la plupart des sujets, et des niveaux intervertébraux, la modélisation adoptée semble tout à fait appropriée : les coefficients de corrélation sont le plus souvent supérieurs à 0,8. Cependant, il existe des niveaux pour lesquels la modélisation est incorrecte. Ce phénomène correspond à des niveaux bloqués ou presque bloqués chez le patient. On considère donc qu'il reste correct de construire la régression linéaire, et les butées contraindront de ce fait suffisamment le modèle.

Cette méthode permet de définir des lois de comportement du disque T1/T2 à L5/S1.

Les centres de rotations d'une vertèbre par rapport à une autre se calculent à partir des différentes positions enregistrées dans le plan frontal (bending) et sagittal (Flexion/extension). Les points de la vertèbre supérieure correspondant à chaque position sont placés dans le repère de Stockes de la vertèbre inférieure. Ces points forment alors une trajectoire considérée comme circulaire dont on calcule le centre de rotation par la méthode des moindres carrés.

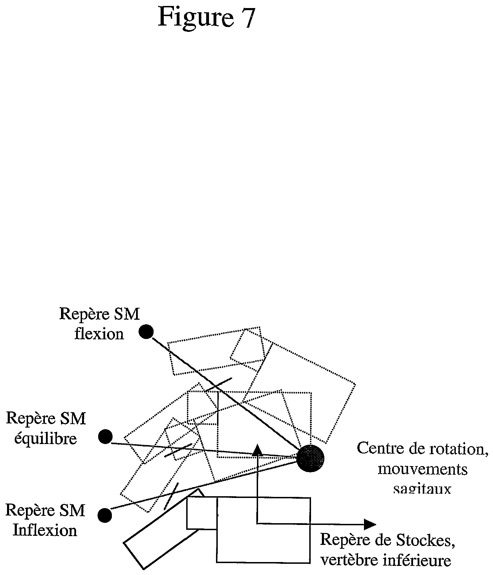

Ceci est illustré sur la figure 7.

Cette méthode permet d'identifier des centres de rotation apparents et personnalisés vérifiant les données expérimentales obtenues sur un patient.

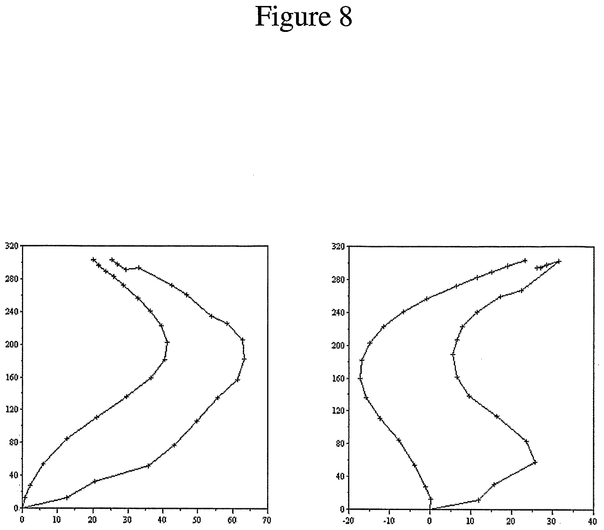

Un exemple de calcul des centres de rotation sur un sujet scoliotique est illustré sur la figure 8.

On constate que cette méthode permet de retrouver la lordose lombaire à partir des acquisitions externes. Cette méthode nécessite un minimum de trois acquisitions distinctes.

Ces centres de rotations sont calculés dans le repère global de la radio. Pour chaque vertèbre, deux centres de rotation distincts sont calculés dans l'espace, un pour les mouvements sagittaux et un pour les mouvements frontaux. Ces centres sont ensuite exprimés dans le repère de Stokes de la vertèbre inférieure de manière à assurer le repositionnement dans le solver.

Un exemple d'algorithme est représenté sur la page suivante.
- Acquisition du modèle cinématique :
   Modèle géométrique issue des radios
   Relation linéaire entre les différents angles intervertébraux
   Amplitude maximum des différents angles intervertébraux
   Position dans les repères de Stokes des vertèbres des centres de rotation frontaux et sagitta
- Initialisation du variant : angle intervertébral L5/S1
- Calcul des angles intervertébraux initiaux sur le modèle géométrique

*Tant que* le **critère de confort** n'est pas optimisé
*Faire*

L'angle variant est incrémenté du pas de calcul (par défaut 0,001)

*Si* l'angle intervertébral variant dépasse ses butées *Alors* l'angle vaut la valeur de la butée et le variant est l'angle suivant

*Pour* tous les angles suivants
*Faire*

L'angle intervertébral i est incrémenté en fonction de l'angle i+1

*Si* l'angle intervertébral i dépasse ses butées *Alors* l'angle vaut la valeur de la butée
*Fin Faire*

Repositionnement du modèle à partir des nouveaux angles intervertébraux.
Application du **critère mécanique** pour repositionnement du modèle autour de l'axe des têtes fémorales
Recalcule des angles intervertébraux sur le modèle géométrique suite à sa déformation Calcul du critère de confort
*Si* le niveau de confort diminue, on incrémente le modèle dans l'autre sens.
Si les deux sens de parcours sont faits, on diminue le niveau d'exigence du critère de confort
*Fin Faire*

La colonne vertébrale est jusqu'à maintenant simulée cinématiquement. On aboutit à une géométrie du rachis représentant l'équilibre du patient. Il est intéressant désormais de connaître la répartition des efforts qui s'exercent sur la colonne, afin de construire un modèle mécanique complet. L'art antérieur connaît une approche pour le calcul des efforts s'inspirant du modèle anthropométrique. Le principe de cette approche est de découper le tronc en quatre tranches rattachées à quatre parties bien précises de la colonne vertébrale et de calculer leur poids et centre de gravité. Le contour de chaque tranche est obtenu par dimensionnement de patron générique de la population moyenne aux dimensions du sujet mesuré.

La méthode utilisée dans le modèle selon l'invention est sensiblement différente. Cinq couronnes sont enregistrées à partir d'enregistrements de contours. Ces couronnes sont ensuite discrétisées en 60 points. Dans un deuxième temps, ces couronnes sont positionnées par rapport à la colonne à partir du recalage. Ce positionnement se fait en confondant le point sur la peau, au niveau de la vertèbre associée à la couronne, enregistré par la courbe de recalage avec le point de la couronne correspondant au centre du dos. Les autres couronnes sont ensuite interpolées pour obtenir 18 couronnes définissant 17 tranches centrées verticalement sur le centre du corps vertébral de la vertèbre correspondante.

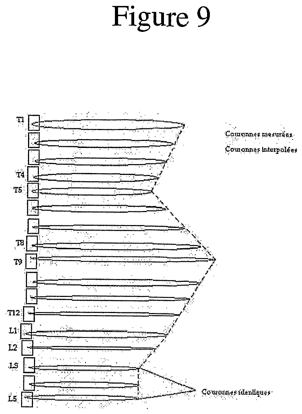

La Figure 9 illustre l'interpolation des couronnes.
Le volume et le centre de gravité de chaque tranche ainsi définie est calculé en les découpant en prismes élémentaires.

A partir des volumes et des centres de gravités de chaque tranche, on dresse le modèle suivant :
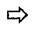 Le poids de la tête et des bras est repris uniquement par la colonne vertébrale
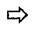 Chaque tranche est divisée en deux : les viscères, et les parties solides comme les cotes, muscles, peau etc....
   ∘ La fraction solide de la tranche est reprise entièrement par la colonne
   ∘ La fraction viscère a un comportement hydrostatique :
      ▪ Les viscères n'engendrent pas de moment
      ▪ Seule la composante normale à la colonne vertébrale voit les efforts transmis par les viscères
      ▪ Les composantes tangentielles à la colonne sont transmises à la tranche d'au-dessous

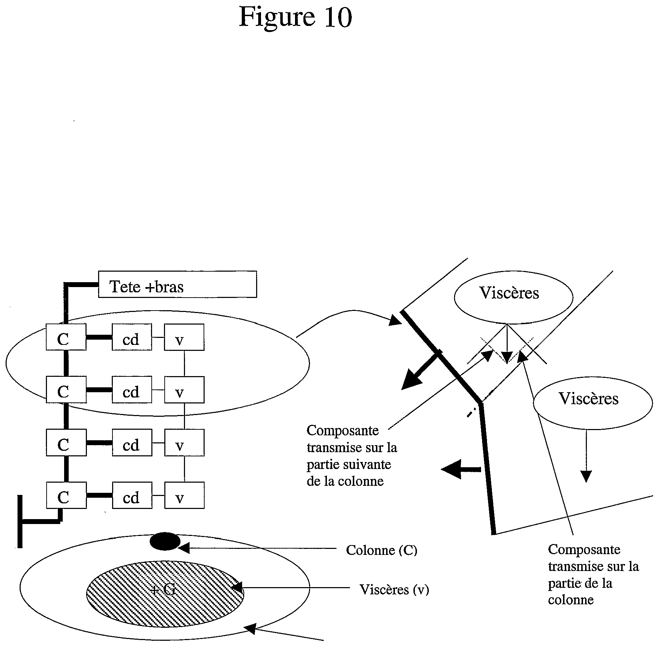

La Figure 10 représente un modèle de répartition des masses dans le tronc.
On obtient alors le torseur des efforts sur chaque vertèbre dépendant uniquement de la géométrie entrée.
La répartition viscère / corps dur pour chaque tranche est pour l'instant défini à partir d'un nombre restreint de données. Cette répartition sera par la suite affinée.

L'architecture du programme se base sur une structure modulaire correspondant à chaque étape de calcul indépendant, de manière à pouvoir faire évoluer le programme sans altérer son fonctionnement. Les modules de calcul sont présentés ci-dessous. Ils sont classés en 4 catégories :
- Les modules d'acquisitions
- Les modules de définition du modèle cinématique
- Les modules du solveur de l'équilibre
- Module de calcul des efforts sur le rachis instrumenté.

L'invention est décrite dans ce qui précède à titre d'exemple. Il est entendu que l'homme du métier est à même de réaliser différentes variantes de l'invention sans pour autant sortir du cadre du brevet.

## Revendications

1. Procédé de construction d'un modèle numérique d'un ensemble d'articulations osseuses, chaque articulation osseuse comportant deux os reliés par une articulation, d'un patient donné, assisté par un ordinateur et dans lequel :
- l'ordinateur comporte au moins un modèle numérique tridimensionnel de référence de l'ensemble d'articulations osseuses, ledit modèle de référence étant représenté, au moins en partie, par des corps rigides modélisant les os et reliés par des articulations dans une position de référence ;
- la géométrie dudit modèle numérique, correspondant à la position relative dans l'espace de chacun des corps rigides, est personnalisée par des données géométriques spécifiques issues directement ou indirectement d'images médicales du patient dans ladite position de référence ;
- ledit modèle numérique est personnalisé mécaniquement par des modifications de paramètres d'interaction, de mobilité ou de rigidité, de chacune des articulations reliant les corps rigides en fonction de caractéristiques constatées sur le patient ;
la personnalisation mécanique du modèle numérique consistant à :
- acquérir les positions dans l'espace d'une partie des corps rigides et déterminer ensuite les positions des autres corps rigides par interpolation, de sorte à obtenir les positions relatives de chacun des corps rigides du modèle numérique ;
- mesurer des positions relatives correspondant à une position générale d'équilibre du patient différente de la position de référence et résultant de l'application d'au moins une contrainte déterminée sur ledit patient ;
- calculer, pour chaque couple de corps rigides, des fonctions analytiques permettant d'approximer les paramètres d'interaction, et qui appliquées au modèle numérique depuis la position de référence conduisent aux positions relatives mesurées correspondant à la position d'équilibre résultant de l'application de l'au moins une contrainte sur le patient.

2. Procédé de construction d'un modèle numérique d'un ensemble d'articulations osseuses selon la revendication 1, **caractérisé en ce que** ledit modèle numérique est défini par des paramètres de positions géométriques des corps rigides et par des paramètres de rigidité des articulations reliant les corps rigides.

3. Procédé de construction d'un modèle numérique d'un ensemble d'articulations osseuses selon la revendication 1 ou 2, **caractérisé en ce qu'**il comporte une étape de représentation du résultat d'une contrainte consistant à recalculer les efforts dans ledit ensemble d'articulations osseuses du modèle personnalisé, en position d'équilibre, résultant d'un ensemble de contraintes simulées, dues à l'implantation d'une prothèse ou d'un implant, comprenant au moins une contrainte statique exercée sur au moins deux corps rigides, et imposant un positionnement relatif avec une mobilité ou une raideur différente de celle correspondant à la loi de comportement.

4. Procédé de construction d'un modèle numérique d'un ensemble d'articulations osseuses selon les revendications 1 et 2, **caractérisé en ce qu'**il comporte une étape d'enregistrement du modèle numérique de référence de l'ensemble d'articulations consistant à définir une alternance de corps rigides et d'articulations, et à définir pour chacun des couples de corps un ensemble de paramètres numériques caractérisant la mobilité ou la raideur globale résultant de l'action de l'ensemble des éléments intercalaires, les disques intervertébraux, et des éléments de liaison, les ligaments, ayant un effet sur les paramètres d'interaction de rigidité entre les deux corps.

5. Procédé de construction d'un modèle numérique d'un ensemble d'articulations osseuses selon l'une au moins des revendications précédentes, **caractérisé en ce que** l'étape de personnalisation consiste à acquérir au moins une image de l'ensemble d'articulations d'un patient donné, à extraire de ladite image les informations nécessaires à la construction d'un modèle réel par reconnaissance de la position des articulations visibles dans ladite image, et de modifier le modèle standard en fonction dudit modèle réel.

6. Procédé de construction d'un modèle numérique d'un ensemble d'articulations osseuses selon l'une au moins des revendications précédentes, **caractérisé en ce que** l'étape d'enregistrement dudit modèle numérique consiste à définir un ensemble standard de données numériques comprenant pour chacune des articulations représentées sous la forme d'un corps rigide :
- un premier descripteur géométrique de position de référence, correspondant à la géométrie de l'ensemble d'articulations pour un patient standard dans une position de référence, ledit descripteur étant déterminé pour chaque corps rigide de façon relative par rapport à un corps adjacent ;
- un deuxième descripteur mécanique d'interaction avec chacun des corps adjacents, ledit descripteur mécanique étant représentatif de la loi de comportement lorsque l'ensemble d'articulations est soumis à l'action d'au moins une contrainte extérieure ;
l'étape de personnalisation consistant à modifier ledit ensemble standard de données par des données personnalisées.

7. Procédé de construction d'un modèle numérique d'un ensemble d'articulations osseuses selon l'une au moins des revendications précédentes, **caractérisé en ce qu'**il comporte en outre une étape de recalage consistant à mettre en correspondance des données d'images radios et des données d'acquisition externe, cette étape se décomposant en deux sous-étapes :
- recalage de la reconstruction radio par rapport à la courbe 3D issue de données d'acquisition externe dans la même position ;
- détermination de la répartition des points de la courbe 3D associés aux vertèbres, positionnés dans le repère de Stokes et leur tangente associée.

## Patentansprüche

1. Verfahren zum Aufbauen eines digitalen Modells einer Einheit von Knochengelenken, wobei jedes Knochengelenk zwei Knochen, die durch ein Gelenk verbunden sind, eines gegebenen Patienten umfasst, das computergestützt ist und wobei:
- der Computer mindestens ein dreidimensionales digitales Bezugsmodell der Einheit von Knochengelenken umfasst, wobei das Bezugsmodell mindestens zum Teil durch starre Körper dargestellt ist, die die Knochen modellieren und durch Gelenke in einer Bezugsposition verbunden sind;
- die Geometrie des digitalen Modells, die der relativen Position in dem Raum jedes der starren Körper entspricht, durch spezifische geometrische Daten personalisiert ist, die direkt oder indirekt aus medizinischen Bildern des Patienten in der Bezugsposition stammen;
- das digitale Modell mechanisch durch Änderungen von Parametern von Wechselwirkung, Mobilität oder Steifigkeit jedes der Gelenke, die die starren Körper verbinden, in Abhängigkeit von Merkmalen, die auf dem Patienten festgestellt werden, personalisiert wird;
wobei die mechanische Personalisierung des digitalen Modells darin besteht:
- Positionen in dem Raum eines Teils der starren Körper zu erfassen und anschließend die Positionen der anderen starren Körper durch Interpolation derart zu bestimmen, dass die relativen Positionen jedes der starren Körper des digitalen Modells erhalten werden;
- die relativen Positionen, die einer allgemeinen Gleichgewichtsposition des Patienten entsprechen, die von der Bezugsposition unterschiedlich ist und aus der Anwendung mindestens einer bestimmten Belastung auf dem Patienten resultiert, zu messen;
- für jedes Paar starrer Körper analytische Funktionen zu berechnen, die es erlauben, die Wechselwirkungsparameter zu approximieren, und die, an das digitale Modell angewandt, ausgehend von der Bezugsposition zu den gemessenen relativen Positionen führen, die der Gleichgewichtsposition entsprechen, die aus dem Anwenden der mindestens einen Belastung auf dem Patienten resultiert.

2. Verfahren zum Aufbauen eines digitalen Modells einer Einheit von Knochengelenken nach Anspruch 1, **dadurch gekennzeichnet, dass** das digitale Modell durch geometrische Positionsparameter der starren Körper und durch Steifigkeitsparameter der Gelenke, die die starren Körper verbinden, definiert ist.

3. Verfahren zum Aufbauen eines digitalen Modells einer Einheit von Knochengelenken nach Anspruch 1 oder 2, **dadurch gekennzeichnet, dass** es einen Darstellungsschritt des Resultats einer Belastung umfasst, der darin besteht, die Kräfte in der Einheit von Knochengelenken des personalisierten Modells in Gleichgewichtsposition, die aus einer Einheit simulierter Belastungen, die auf das Implantieren einer Prothese oder eines Implantats zurückzuführen sind, resultieren, die mindestens eine statische Belastung umfasst, die auf mindestens zwei starre Körper ausgeübt wird und eine relative Positionierung mit einer unterschiedlichen Mobilität oder Steifigkeit von der, die dem Verhaltensgesetz entspricht, neu zu berechnen.

4. Verfahren zum Aufbauen eines digitalen Modells einer Einheit von Knochengelenken nach den Ansprüchen 1 und 2, **dadurch gekennzeichnet, dass** es einen Aufzeichnungsschritt des digitalen Bezugsmodells der Einheit von Gelenken umfasst, der darin besteht, ein Abwechseln starrer Körper und Gelenke zu definieren, und für jedes der Paare von Körpern eine Einheit digitaler Parameter zu definieren, die die Mobilität oder Steifigkeit insgesamt charakterisieren, die aus der Aktion der Einheit von Zwischenelementen, den Bandscheiben und Verbindungselementen, den Ligamenten, die sich auf die Wechselwirkungsparameter der Steifigkeit zwischen den zwei Körpern auswirken, resultieren.

5. Verfahren zum Aufbauen eines digitalen Modells einer Einheit von Knochengelenken nach mindestens einem der vorstehenden Ansprüche, **dadurch gekennzeichnet, dass** der Personalisierungsschritt darin besteht, mindestens ein Bild der Einheit von Gelenken eines gegebenen Patienten zu erfassen, aus dem Bild die Informationen zu extrahieren, die für das Aufbauen eines realen Modells durch Erkennen der Position der Gelenke, die in dem Bild sichtbar sind, erforderlich sind, und das Standardmodell in Abhängigkeit von dem realen Modell zu modifizieren.

6. Verfahren zum Aufbauen eines digitalen Modells einer Einheit von Knochengelenken nach mindestens einem der vorstehenden Ansprüche, **dadurch gekennzeichnet, dass** der Aufzeichnungsschritt des digitalen Modells darin besteht, eine Standardeinheit digitaler Daten zu definieren, die für jedes der dargestellten Gelenke in Form eines starren Körpers Folgendes umfasst:
- einen ersten geometrischen Bezugspositionsdeskriptor, der der Geometrie der Einheit von Gelenken für einen Standardpatienten in einer Bezugsposition entspricht, wobei der Deskriptor für jeden starren Körper bezüglich einem benachbarten Körper bestimmt wird;
- einen zweiten mechanischen Wechselwirkungsdeskriptor mit jedem der benachbarten Körper, wobei der mechanische Deskriptor für das Verhaltensgesetz repräsentativ ist, wenn die Gruppe von Gelenken der Wirkung mindestens einer äußeren Belastung unterzogen wird;
wobei der Personalisierungsschritt darin besteht, die Standardeinheit von Daten durch personalisierte Daten zu ändern.

7. Verfahren zum Aufbauen eines digitalen Modells einer Einheit von Knochengelenken nach mindestens einem der vorstehenden Ansprüche, **dadurch gekennzeichnet, dass** es außerdem einen Abstimmungsschritt umfasst, der darin bestimmt, die Röntgenbilddaten und Daten externer Erfassung in Übereinstimmung zu bringen, wobei dieser Schritt in zwei Unterschritte gegliedert ist:
- Abstimmen der Röntgenrekonstruktion bezüglich der 3D-Kurve, die aus den Daten externer Erfassung in derselben Position hervorgehen;
- Bestimmen der Verteilung der Punkte der 3D-Kurve, die mit den Wirbeln assoziiert sind, die in der Stokes-Kennzeichnung positioniert sind, und ihrer dazugehörenden Tangente.

## Claims

1. Method for constructing a digital model of a set of osseous joints, each osseous joint including two bones connected by a joint, of a given patient, assisted by a computer and wherein:
- the computer includes at least one reference three-dimensional digital model of the set of osseous joints, said reference model being represented, at least in part, by rigid bodies modelling the bones and connected by joints in a reference position;
- the geometry of said digital model, corresponding to the relative spatial position of each of the rigid bodies, is customised by specific geometric data derived directly or indirectly from medical images of the patient in said reference position;
- said digital model is customised mechanically by modifications of interaction, mobility or rigidity parameters, of each of the joints connecting the rigid bodies according to characteristics observed on the patient;
the mechanical customisation of the digital model consisting of:
- acquiring the spatial positions of a portion of the rigid bodies and subsequently determining the positions of the other rigid bodies by interpolation, so as to obtain the relative positions of each of the rigid bodies of the digital model;
- measuring relative positions corresponding to a general equilibrium position of the patient different from the reference position and resulting from the application of at least one determined strain on said patient;
- computing, for each pair of rigid bodies, analytical functions for approximating the interaction parameters, and which applied to the digital model from the reference position lead to measured relative positions corresponding to the resultant equilibrium position from the application of the at least one strain on the patient.

2. Method for constructing a digital model of a set of osseous joints according to claim 1, **characterised in that** said digital model is defined by geometric position parameters of the rigid bodies and by rigidity parameters of the joints connecting the rigid bodies.

3. Method for constructing a digital model of a set of osseous joints according to claim 1 or 2, **characterised in that** it includes a step of representing the result of a strain consisting of recomputing the stress in said set of osseous joints of the customised model, in the equilibrium position, resulting from a set of simulated strains, due to the implantation of a prosthesis or an implant, comprising at least one static strain exerted on at least two rigid bodies, and imposing a relative positioning with a mobility or a stiffness different from that corresponding to the behaviour law.

4. Method for constructing a digital model of a set of osseous joints according to claims 1 and 2, **characterised in that** it includes a step of recording the reference digital model of the set of joints consisting of defining an alternation of rigid bodies and joints, and defining for each of the pairs of bodies a set of digital parameters characterising the mobility or the overall stiffness resulting from the action of the set of intercalated elements, the intervertebral discs, and the linking elements, the ligaments, having an effect on the rigidity interaction parameters between the two bodies.

5. Method for constructing a digital model of a set of osseous joints according to at least one of the preceding claims, **characterised in that** the customisation step consists of acquiring at least one image of the set of joints of a given patient, extracting from said image the information required for the construction of a real model by recognition of the position of the joints visible in said image, and modifying the standard model according to said real model.

6. Method for constructing a digital model of a set of osseous joints according to at least one of the preceding claims, **characterised in that** the step of recording said digital model consists of defining a standard set of digital data comprising for each of the joints represented in the form of a rigid body:
- a first geometric descriptor of reference position, corresponding to the geometry of the set of joints for a standard patient in a reference position, said descriptor being determined for each rigid body in a relative manner with respect to an adjacent body;
- a second mechanical descriptor of interaction with each of the adjacent bodies, said mechanical descriptor being representative of the behaviour law when the set of joints is subjected to the action of at least one external strain;
the customisation step consisting of modifying said standard set of data by customised data.

7. Method for constructing a digital model of a set of osseous joints according to at least one of the preceding claims, **characterised in that** it further includes a realignment step consisting of matching X-ray image data and external acquisition data, this step being broken down into two substeps:
- realignment of the X-ray reconstruction with respect to the 3D curve derived from external acquisition data in the same position;
- determination of the distribution of the points of the 3D curve associated with the vertebrae, positioned in the Stokes reference frame and the associated tangent thereof.
